(19) **Europäisches Patentamt / European Patent Office / Office européen des brevets**

(11) **EP 3 197 366 B1**

(12) **EUROPEAN PATENT SPECIFICATION**

(45) Date of publication and mention of the grant of the patent:
**02.10.2019  Bulletin 2019/40**

(21) Application number: **15844503.1**

(22) Date of filing: **02.01.2015**

(51) Int Cl.:
*A61B 8/00* (2006.01)     *A61B 8/08* (2006.01)
*A61B 8/14* (2006.01)     *G01S 7/52* (2006.01)

(86) International application number:
**PCT/KR2015/000006**

(87) International publication number:
**WO 2016/047867 (31.03.2016 Gazette 2016/13)**

(54) **ULTRASOUND IMAGE PROCESSING METHOD AND ULTRASOUND IMAGING APPARATUS THEREOF**

ULTRASCHALLBILDVERARBEITUNGSVERFAHREN UND ULTRASCHALLBILDGEBUNGSVORRICHTUNG DAFÜR

PROCÉDÉ DE TRAITEMENT D'IMAGE À ULTRASONS ET APPAREIL D'IMAGERIE À ULTRASONS ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **25.09.2014  KR 20140128281**

(43) Date of publication of application:
**02.08.2017  Bulletin 2017/31**

(73) Proprietor: **Samsung Electronics Co., Ltd. Gyeonggi-do 16677 (KR)**

(72) Inventors:
• **LEE, Hyoung-ki**
  **Seongnam-si**
  **Gyeonggi-do 463-882 (KR)**
• **KONG, Dong-geon**
  **Hwaseong-si**
  **Gyeonggi-do 445-792 (KR)**

• **PARK, Jun-ho**
  **Hwaseong-si**
  **Gyeonggi-do 445-776 (KR)**
• **CHOI, Ki-wan**
  **Anyang-si**
  **Gyeonggi-do 431-820 (KR)**

(74) Representative: **D'Halleweyn, Nele Veerle Trees Gertrudis et al**
  **Arnold & Siedsma**
  **Bezuidenhoutseweg 57**
  **2594 AC The Hague (NL)**

(56) References cited:
**WO-A1-2014/128084     US-A1- 2006 173 319
US-A1- 2007 093 716     US-A1- 2007 093 716
US-A1- 2009 216 119     US-A1- 2009 216 119
US-A1- 2013 046 175     US-A1- 2013 317 361
US-B1- 6 770 033        US-B1- 6 770 033**

## Description

## Technical Field

One or more exemplary embodiments relate to methods of measuring a stress applied to a tissue and generating an elasticity image based on the measured stress, and ultrasound imaging apparatuses thereof.

## Background Art

An ultrasound imaging apparatus irradiates ultrasound signals generated by transducers of a probe to an object and receives echo signals reflected from the object, thereby obtaining images regarding the interior of the object (e.g., tomography of soft tissues or blood flow). In particular, an ultrasound imaging apparatus may be used for medical purposes including observation of the interior of an object, detection of foreign substances, and diagnosis of damage. The ultrasound imaging apparatus may display information regarding an object in real time. Furthermore, unlike the use of X-rays, use of the ultrasound imaging apparatus is very safe as it does not involve any radioactive exposure. Therefore, the ultrasound imaging apparatus is widely used together with other types of imaging apparatuses such as computer tomography (CT) scanners, magnetic resonance imaging (MRI) apparatuses, and nuclear medical diagnosis apparatuses.

A tumor generated in a soft tissue such as a breast or a prostate is harder than its surroundings. Since a tumor tissue is harder than a surrounding tissue, a diagnosing doctor may diagnose the presence/absence of a tumor by directly pressing a tissue.

The ultrasound imaging apparatus may replace palpation. For example, the ultrasound imaging apparatus may transmit an ultrasound signal to a tissue and calculate the hardness of the tissue based on an ultrasound echo signal received from the tissue.

US 2009/0216119 A1 is related to a method for determining shear information with ultrasound. The shear modulus information is measured for sparse locations in a scanning field of view. For other locations, the shear modulus information is calculated as a function of the sparsely measured values and strain information. For example, shear modulus values are provided for every grid point in a field of view based on strain values for every grid point and on sparsely measured shear modulus values.

## Disclosure of Invention

## Solution to Problem

One or more exemplary embodiments include methods of measuring a stress applied to a tissue and generating an elasticity image based on the measured stress, and ultrasound imaging apparatuses thereof.

The invention is defined in claim 1 and claim 7. Further aspects and preferred embodiments are defined in the appended claims. Aspects, embodiments and examples of the present disclosure which do not fall under the scope of the appended claims do not form part of the invention and are merely provided for illustrative purposes.

Additional aspects will be set forth in part in the description which follows and, in part, will be apparent from the description, or may be learned by practice of the presented exemplary embodiments.

## Advantageous Effects of Invention

One or more exemplary embodiments include methods of generating an elasticity image more accurately.

## Brief Description of Drawings

These and/or other aspects will become apparent and more readily appreciated from the following description of exemplary embodiments, taken in conjunction with the accompanying drawings in which:

FIG. 1 is a block diagram illustrating a configuration of an ultrasound imaging apparatus according to an exemplary embodiment;
FIG. 2 is a flowchart illustrating a method of calculating, by an ultrasound imaging apparatus, a stress applied to at least one point in a region of interest (ROI), according to an exemplary embodiment;
FIG. 3 is a diagram illustrating a method of calculating, by an ultrasound imaging apparatus, a shear modulus of at least one point in an ROI, according to an exemplary embodiment;
FIG. 4 is a diagram illustrating a method of calculating, by an ultrasound imaging apparatus, a shear modulus of an ROI, based on a propagation velocity of a shear wave in the ROI, according to an exemplary embodiment;
FIG. 5 is a diagram illustrating a method of calculating, by an ultrasound imaging apparatus, a strain of an ROI, according to an exemplary embodiment;
FIGS. 6a and 6b are diagrams illustrating a strain of a tissue generated by a force applied manually through a probe of an ultrasound imaging apparatus, according to an exemplary embodiment;
FIG. 7 is a diagram illustrating a method of calculating, by an ultrasound imaging apparatus, a strain generated by a force applied manually through a probe, according to an exemplary embodiment;
FIG. 8 is a diagram illustrating a method of calculating, by an ultrasound imaging apparatus, a stress of an ROI, based on a shear modulus and a strain, according to an exemplary embodiment;
FIG. 9 is a flowchart illustrating a method of guiding, by an ultrasound imaging apparatus, an operation of a user pressing a probe, according to an exemplary embodiment;

FIG. 10 is a diagram illustrating a method of calculating, by an ultrasound imaging apparatus, a force of a user applied to an object through a probe, according to an exemplary embodiment;

FIG. 11 is a diagram illustrating a method of guiding, by an ultrasound imaging apparatus, an operation of a user pressing a probe when capturing a shear modulus image, according to an exemplary embodiment;

FIG. 12 is a diagram illustrating a method of guiding, by an ultrasound imaging apparatus, an operation of a user pressing a probe when capturing a shear modulus image, according to an exemplary embodiment;

FIG. 13 is a flowchart illustrating a method of recalculating, by an ultrasound imaging apparatus, a strain by using a stress, according to an exemplary embodiment;

FIG. 14 is a diagram illustrating a method of recalculating, by an ultrasound imaging apparatus, a strain by using a stress, according to an exemplary embodiment;

FIG. 15 is a diagram illustrating a method of recalculating, by an ultrasound imaging apparatus, a strain by using a stress, according to another exemplary embodiment;

FIGS. 16a and 16b are diagrams illustrating a method of displaying, by an ultrasound imaging apparatus, a shear modulus image and a strain image, according to an exemplary embodiment; and

FIG. 17 is a block diagram illustrating a configuration of an ultrasound imaging apparatus according to an exemplary embodiment.

**Best Mode for Carrying out the Invention**

According to one or more exemplary embodiments, an ultrasound imaging apparatus includes: a data acquiring unit transmitting an ultrasound signal to a region of interest (ROI) of an object and then receiving an ultrasound echo signal reflected from the ROI of the object; and a control unit calculating a shear modulus of at least one point in the ROI and a strain of the at least one point, based on the received ultrasound echo signal, and calculating a stress applied to the at least one point by using the shear modulus and the strain.

The control unit may generate first information representing a compression stress applied to the object, based on the stress, and the ultrasound imaging apparatus further includes a display unit displaying a user interface screen including the first information under the control of the control unit.

The control unit may recalculate the strain, based on a value obtained by dividing the strain by the stress, and form a strain image of the ROI by using the recalculated strain.

The control unit may divide the ROI into at least one region, calculate an average stress of each of the at least one region, and recalculate the strain, based on a value obtained by dividing the strain by the average stress.

The control unit may calculate a value, which is proportional to a product of the shear modulus and the strain, as the stress.

The control unit may acquire a shear modulus map of the ROI by using the shear modulus of the at least one point, acquire a strain imaging map of the ROI by using the strain of the at least one point, and calculate the stress of the ROI, based on the shear modulus map and the strain imaging map.

The ultrasound signal may include a first ultrasound signal used to generate a reference image, a second ultrasound signal that is a focused ultrasonic pulse used to apply an acoustic force to the ROI, and a third ultrasound signal used to calculate a displacement of the at least one point in the ROI, which is generated by the acoustic force and a pressure applied through an ultrasound probe, and the ultrasound echo signal may include a first ultrasound echo signal corresponding to the first ultrasound signal and a third ultrasound echo signal corresponding to the third ultrasound signal, which are reflected from the ROI of the object.

The control unit may include a shear modulus calculating module calculating a time-dependent displacement of the at least one point, based on the third ultrasound echo signal, and calculating the shear modulus of the at least one point by using the time-dependent displacement.

The control unit may include a strain calculating module acquiring reference image data of the ROI by using the first ultrasound echo signal, calculating a displacement of the at least one point in the ROI, which is generated by a pressure applied through the ultrasound probe, based on the reference image data and the third ultrasound echo signal, and calculating the strain of the at least one point in the ROI by differentiating the calculated displacement.

The control unit may generate a shear modulus image of the ROI by using the shear modulus of the at least one point and generates a strain image of the ROI by using the strain of the at least one point, and the ultrasound imaging apparatus may further include a display unit displaying the shear modulus image and the strain image simultaneously on a screen.

According to one or more exemplary embodiments, an ultrasound image processing method includes: transmitting an ultrasound signal to a region of interest (ROI) of an object and then receiving an ultrasound echo signal reflected from the ROI of the object; calculating a shear modulus of at least one point in the ROI, based on the received ultrasound echo signal; calculating a strain of the at least one point, based on the received ultrasound echo signal; and calculating a stress applied to the at least one point by using the shear modulus and the strain.

The ultrasound image processing method may further include: generating first information representing a compression stress applied to the object, based on the stress; and displaying a user interface screen including the first information.

The ultrasound image processing method may further include: recalculating the strain, based on a value obtained by dividing the strain by the stress; and forming a strain image of the ROI by using the recalculated strain.

The recalculating of the strain based on the value obtained by dividing the strain by the stress may include dividing the ROI into at least one region, calculating an average stress of each of the at least one region, and recalculating the strain, based on a value obtained by dividing the strain by the average stress.

The calculating of the stress applied to the at least one point by using the shear modulus and the strain may include calculating a value, which is proportional to a product of the shear modulus and the strain, as the stress.

The calculating of the stress applied to the at least one point by using the shear modulus and the strain may include acquiring a shear modulus map of the ROI by using the shear modulus of the at least one point, acquiring a strain imaging map of the ROI by using the strain of the at least one point, and calculating the stress of the ROI, based on the shear modulus map and the strain imaging map.

The ultrasound signal may include a first ultrasound signal used to generate a reference image, a second ultrasound signal that is a focused ultrasonic pulse used to apply an acoustic force to the ROI, and a third ultrasound signal used to calculate a displacement of the at least one point in the ROI, which is generated by the acoustic force and a pressure applied through an ultrasound probe, and the ultrasound echo signal may include a first ultrasound echo signal corresponding to the first ultrasound signal and a third ultrasound echo signal corresponding to the third ultrasound signal, which are reflected from the ROI of the object.

The calculating of the shear modulus of the at least one point may include: calculating a time-dependent displacement of the at least one point, based on the third ultrasound echo signal; and calculating the shear modulus of the at least one point by using the time-dependent displacement.

The calculating of the strain of the at least one point may include: acquiring reference image data of the ROI by using the first ultrasound echo signal; calculating a displacement of the at least one point in the ROI, which is generated by a pressure applied through the ultrasound probe, based on the reference image data and the third ultrasound echo signal; and calculating the strain of the at least one point in the ROI by differentiating the calculated displacement.

The ultrasound image processing method may further include: generating a shear modulus image of the ROI by using the shear modulus of the at least one point; generating a strain image of the ROI by using the strain of the at least one point; and displaying the shear modulus image and the strain image simultaneously on a screen.

## Mode for the Invention

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects of the present description. As used herein, expressions such as "at least one of," when preceding a list of elements, modify the entire list of elements and do not modify the individual elements of the list.

The terms used in the specification will be briefly described, and the exemplary embodiments will be described in detail.

The terms used in this specification are those general terms currently widely used in the art in consideration of functions in regard to the exemplary embodiments, but the terms may vary according to the intention of those of ordinary skill in the art, precedents, or new technology in the art. Also, specified terms may be selected by the applicant, and in this case, the detailed meaning thereof will be described in the detailed description of the exemplary embodiments. Thus, the terms used in the specification should be understood not as simple names but based on the meaning of the terms and the overall description of the exemplary embodiments.

When something "comprises" or "includes" a component, another component may be further included unless specified otherwise. Also, the terms "units" and "modules" used herein refer to units that perform at least one function or operation, and the units may be implemented as hardware or software or a combination of hardware and software.

Throughout the specification, an "ultrasound image" refers to an image of an object, which is acquired by using ultrasonic waves. Also, an "object" may include a person or an animal, or a part of a person or an animal. For example, the object may include organs such as a liver, a heart, a womb, a brain, a breast, and an abdomen, or a blood vessel. Also, the object may include a phantom. The phantom may refer to a material having a volume that is approximately the intensity and effective atomic number of a living thing, and may include a spherical phantom having a property similar to a human body.

Also, a "user" may be, but is not limited to, a medical expert, such as a doctor, a nurse, a medical laboratory technologist, or a medial image expert, or a technician who repairs a medical apparatus.

Hereinafter, exemplary embodiments will be described in detail with reference to the accompanying drawings so that those of ordinary skill in the art may easily implement the exemplary embodiments. However, the exemplary embodiments may have different forms and should not be construed as being limited to the descriptions set forth herein. In addition, portions irrelevant to the description of the exemplary embodiments will be omitted in the drawings for a clear description of the exemplary embodiments, and like reference numerals will denote like elements throughout the specification.

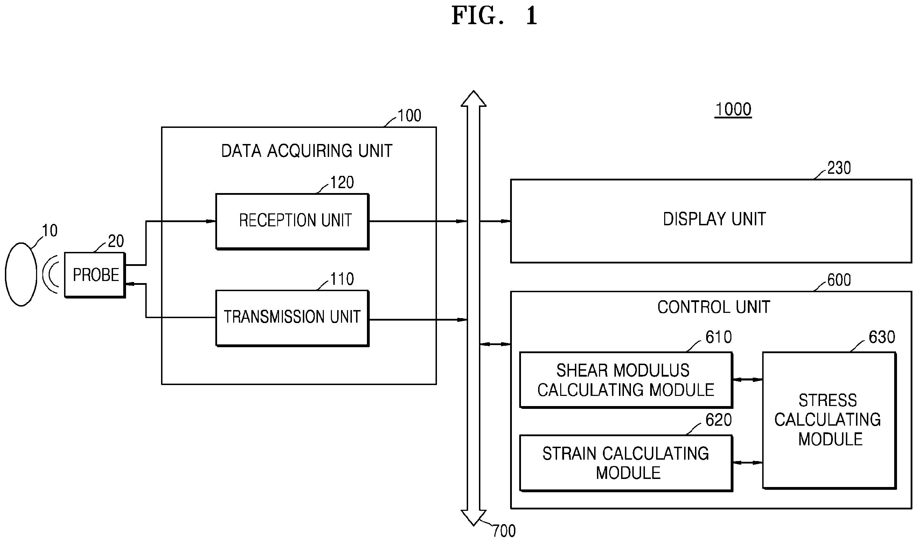

FIG. 1 is a block diagram illustrating a configuration of an ultrasound imaging apparatus 1000 according to an exemplary embodiment.

Referring to FIG. 1, the ultrasound imaging apparatus 1000 may include a display unit 230, a control unit 600, and a data acquiring unit 100. The display unit 230, the control unit 600, and the data acquiring unit 100 may be connected to one another via buses 700. Also, the ultrasound imaging apparatus 1000 may further include a probe (or ultrasound probe) 20 in addition to the display unit 230, the control unit 600, and the data acquiring unit 100.

The probe 20 may transmit an ultrasound signal to an object 10 according to a driving signal applied from the data acquiring unit 100 and receive an echo signal reflected from the object 10. The probe 20 may include a plurality of transducers, and the plurality of transducers may oscillate according to an electrical signal transmitted thereto and generate an ultrasonic wave, that is, acoustic energy. Also, the probe 20 may be connected to a main body of the ultrasound imaging apparatus 1000 by wire or wirelessly. According to exemplary embodiments, the ultrasound imaging apparatus 1000 may include a plurality of probes 20.

The data acquiring unit 100 may include a transmission unit 110 and a reception unit 120. The transmission unit 110 may supply a driving signal to the probe 20. The reception unit 120 may generate ultrasound data by processing the echo signal received from the probe 20.

In general, the control unit 600 may control overall operations of the ultrasound imaging apparatus 1000. For example, the control unit 600 may control the data acquiring unit 100 and the display unit 230.

The control unit 600 may include a shear modulus calculating module 610, a strain calculating module 620, and a stress calculating module 630.

The shear modulus calculating module 610 may calculate a shear modulus of a region of interest (ROI) of the object 10, based on the ultrasound data generated by the data acquiring unit 100. The shear modulus may refer to a modulus that represents elasticity of an object with respect to one face of the object.

The strain calculating module 620 may calculate a strain generated by a force applied to the ROI of the object 10, based on the ultrasound data generated by the data acquiring unit 100. The strain may refer to a variation of an object per unit length.

Also, the stress calculating module 630 may calculate a stress applied to the ROI of the object 10, based on the shear modulus calculated by the shear modulus calculating module 610 and the strain calculated by the strain calculating module 620. For example, the stress calculating module 630 may calculate a value, which is proportional to a product of the shear modulus and the strain, as the stress.

Also, the strain calculating module 620 may recalculate the strain of the ROI of the object 10, based on the calculated stress. For example, the strain calculating module 620 may recalculate a value, which is proportional to a value obtained by dividing the precalculated strain by the calculated stress, as the strain.

The display unit 230 may generate an ultrasound image by scan-converting the ultrasound data generated by the data acquiring unit 100 and display the ultrasound image.

Also, the display unit 230 may display a strain image of the ROI of the object 10 generated based on the strain calculated by the strain calculating module 620. In this case, together with the strain image, the display unit 230 may display the force applied to the ROI of the object 10 by the probe 20. Also, the display unit 230 may display a shear modulus image of the ROI of the object 10 generated based on the shear modulus calculated by the shear modulus calculating module 610.

Also, the display unit 230 may display the shear modulus image and the strain image simultaneously on a screen. Also, the display unit 230 may display the shear modulus image and the strain image in a superimposed manner.

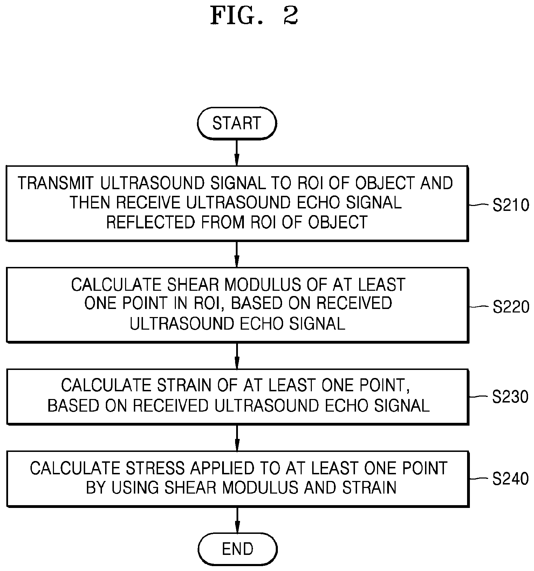

FIG. 2 is a flowchart illustrating a method of calculating, by the ultrasound imaging apparatus 1000, a stress applied to at least one point in the ROI, according to an exemplary embodiment.

Referring to FIG. 2, in operation S210, the ultrasound imaging apparatus 1000 may transmit an ultrasound signal to the ROI of the object 10 and then receive an ultrasound echo signal reflected from the ROI of the object 10.

The ultrasound signal may include a first ultrasound signal used to generate a reference image and a second ultrasound signal that is a focused ultrasonic pulse used to apply an acoustic force to the ROI. Also, the ultrasound signal may include a third ultrasound signal used to calculate a displacement of at least one point in the ROI, which is generated by the acoustic force and a pressure applied through the probe 20.

The ultrasound echo signal may include a first ultrasound echo signal corresponding to the first ultrasound signal and a third ultrasound echo signal corresponding to the third ultrasound signal, which are reflected from the ROI of the object 10.

In operation S220, the ultrasound imaging apparatus 1000 may calculate a shear modulus of the at least one point in the ROI, based on the received ultrasound echo signal.

The ultrasound imaging apparatus 1000 may calculate the shear modulus of the ROI, based on a displacement of a tissue generated by the acoustic force.

For example, the ultrasound imaging apparatus 1000 may calculate a time-dependent displacement of the at least one point, based on the third ultrasound echo signal and calculate the shear modulus of the at least one point by using the time-dependent displacement.

In operation S230, the ultrasound imaging apparatus 1000 may calculate a strain of the at least one point, based on the received ultrasound echo signal.

The ultrasound imaging apparatus 1000 may acquire reference image data of the ROI by using the first ultrasound echo signal, calculate a displacement of the at least one point in the ROI, which is generated by a pressure applied through the probe 20, based on the reference image data and the third ultrasound echo signal, and calculate the strain of the at least one point in the ROI by differentiating the calculated displacement.

In operation S240, the ultrasound imaging apparatus 1000 may calculate a stress applied to the at least one point by using the shear modulus and the strain.

The ultrasound imaging apparatus 1000 may calculate a value, which is proportional to the product of the shear modulus and the strain, as the stress.

Also, the ultrasound imaging apparatus 1000 may acquire a shear modulus map of the ROI by using the shear modulus of the at least one point, acquire a strain imaging map of the ROI by using the strain of the at least one point, and calculate the stress of the ROI, based on the shear modulus map and the strain imaging map.

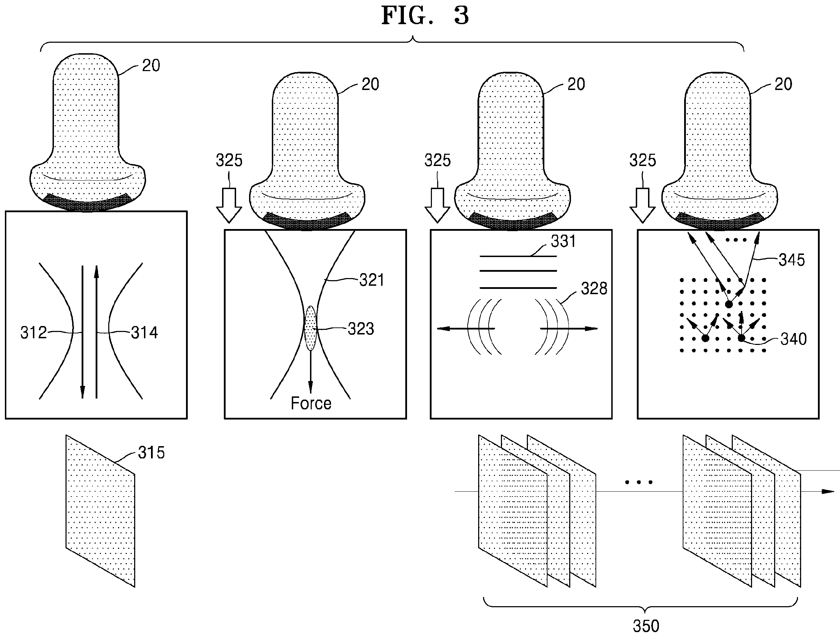

FIG. 3 is a diagram illustrating a method of calculating, by the ultrasound imaging apparatus 1000, a shear modulus of at least one point in the ROI, according to an exemplary embodiment.

Referring to FIG. 3, the control unit 600 may generate a shear modulus image of the ROI. The shear modulus image may be an image that represents a stiffness of the tissue, based on a shear modulus of the tissue located in the ROI.

The data acquiring unit 100 may transmit a first ultrasound signal 312 to the ROI through the probe 20 and receive a first ultrasound echo signal 314 corresponding to the first ultrasound signal 312 from the ROI.

As the first ultrasound echo signal 314 is received, the control unit 600 may generate a reference image 315 of the ROI, based on the first ultrasound echo signal 314. The reference image 315 may be an image that represents a position of the tissue before a force is applied to the ROI. The reference image 315 may be a B mode image or an M mode image of the ROI.

After receiving the first ultrasound echo signal 314, the data acquiring unit 100 may transmit a second ultrasound signal 321 of a focusing point 323 in the ROI through the probe 20. The second ultrasound signal 321 may be a focused ultrasonic pulse.

When a strong focused ultrasonic pulse 321 is transmitted to the focusing point 323 in the ROI, a transverse wave 328 may be generated in the tissue located in the ROI. For example, the strong focused ultrasonic pulse 321 may push the tissue in the direction of an ultrasonic pulse (i.e., axial direction). An axial movement of the tissue located at the focusing point 323 may cause an adjacent tissue to move in the same direction (axial direction). As the tissue adjacent to the focusing point 323 moves in the same direction, the movement may again propagate to a tissue adjacent to the moved tissue. In this case, the force of the ultrasonic wave causing the tissue to move may be referred to as an acoustic force.

As the movement propagates to the adjacent tissue, the acoustic force applied to the focusing point 323 may generate a wave in a direction perpendicular to the direction of the ultrasonic pulse (i.e., lateral direction) with the focusing point 323 being a point of origin. The transverse wave 328 propagating in the direction perpendicular to the direction of the ultrasonic pulse may be referred to as a shear wave.

The propagation velocity of the shear wave 328 may be determined according to the stiffness, Young's modulus, or shear modulus of the tissue.

For example, the propagation velocity of the shear wave 328 may change to about 1 m/s to about 10 m/s according to the stiffness of the tissue. Also, the propagation velocity of the shear wave 328 in the tissue may increase as the stiffness of the tissue increases.

Also, the propagation velocity of the shear wave 328 in the tissue may have a relationship with the stiffness of the tissue as expressed by Equation 1 below.

$$\text{Equation 1}$$
$$G = \rho \cdot C^2$$

In Equation 1, G may denote the stiffness of the tissue, $\rho$ may denote the density of the tissue, and C may denote the propagation velocity of the shear wave 328. The density "$\rho$" of the tissue may be regarded as a constant value in the ROI and may be mostly a known value. Accordingly, the stiffness of the tissue may be detected by measuring the propagation velocity of the shear wave 328 propagating in the tissue.

The shear wave 328 may be detected by measuring the displacement of the tissue in the direction of the ultrasonic pulse (i.e., axial direction). The displacement of the tissue may refer to a movement distance of the tissue in the axial direction with respect to the reference image 315. Also, the propagation velocity of the shear wave 328 at a partial tissue in the ROI may be calculated based on a time point at which the displacement of the partial tissue and the displacement of a tissue adjacent to the partial tissue are at their maximum.

Referring to FIG. 3, in order to calculate the displacement of the tissue generated by the acoustic force, the data acquiring unit 100 may transmit a third ultrasound signal 331 to the ROI. In this case, in order to more accurately measure the propagation velocity of the shear wave 328, the data acquiring unit 100 may transmit a plane wave as the third ultrasound signal 331. When a plane wave is transmitted as the third ultrasound signal 331, the ultrasound imaging apparatus 1000 may image the shear wave 328 at a rate of thousands of frames per second.

After the third ultrasonic pulse 331 is transmitted to the tissue, the third ultrasonic pulse 331 may be scattered by a scatter 340 located at the tissue in the ROI. The third ultrasonic pulse 331 scattered by the scatter 340 may return to the probe 20. In this case, the third ultrasonic pulse 331 scattered by the scatter 340 may be referred to as a third ultrasonic echo pulse 345.

As the third ultrasonic echo pulse 345 is received, the control unit 600 may generate ultrasound images of the ROI, based on the third ultrasonic echo pulse 345. An image including the shear wave 328, from among the ultrasound images generated based on the third ultrasonic echo pulse 345, may be referred to as a shear wave image 350. When a plane wave is transmitted as the third ultrasound signal 331, the control unit 600 may generate the shear wave image 350 at a rate of thousands of frames per second.

The shear wave image 350 may represent the displacement of the tissue in the ROI. The control unit 600 may calculate the propagation velocity of the shear wave 328 by using the displacement of the tissue in the ROI.

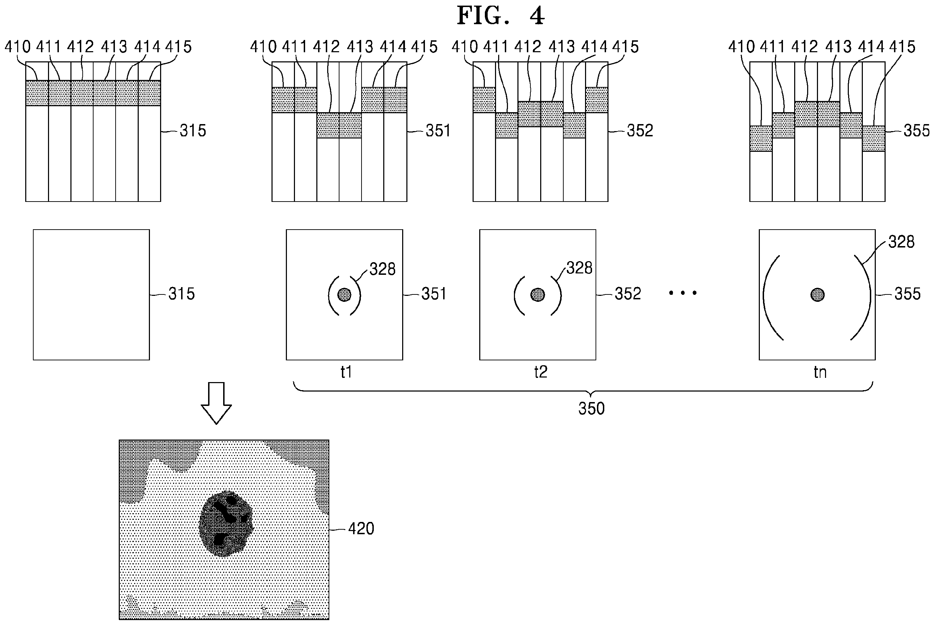

FIG. 4 is a diagram illustrating a method of calculating, by the ultrasound imaging apparatus 1000, a shear modulus of the ROI, based on a propagation velocity of a shear wave in the ROI, according to an exemplary embodiment.

The shear modulus calculating module 610 may calculate a shear modulus of the ROI, based on the displacement of the tissue generated by the acoustic force.

For example, the shear modulus calculating module 610 may detect a moved position of a first partial tissue (e.g., one of 410 to 415) in the reference image 315 from each shear wave image 351, based on a cross-correlation method. Based on the detected position, the shear modulus calculating module 610 may calculate an axial displacement of the first partial tissue (e.g., one of 410 to 415). Based on the calculated displacement, the shear modulus calculating module 610 may detect a time point at which the displacement of the first partial tissue is at its maximum. The shear modulus calculating module 610 may determine the time point at which the displacement of the first partial tissue is at its maximum as a time point at which the shear wave 328 has reached the first partial tissue.

Referring to FIG. 4, the ultrasound data constituting a first image 351 from among a plurality of shear wave images 350 is received at a time point "t1", and the displacements of two partial tissues 412 and 413 adjacent to the focusing point may be at their maximum in the first image 351. Accordingly, the shear modulus calculating module 610 may determine that the shear wave 328 has reached the two partial tissues 412 and 413 at the time point "t1". Also, the shear modulus calculating module 610 may detect the position of the shear wave 328 and the time point the shear wave 328 has arrived with respect to the plurality of shear wave images 350 in the same manner, and may calculate the velocity of the shear wave 328 at the at least one point in the ROI, based on the position of the shear wave 328 and the time point the shear wave 328 has arrived.

As the velocity of the shear wave 328 at the at least one point in the ROI is calculated, the shear modulus calculating module 610 may calculate a shear modulus of a point, at which the velocity of the shear wave 328 has been calculated, by substituting the density of the ROI and the velocity of the shear wave 328 into Equation 1.

As the shear modulus of the at least one point in the ROI is calculated, the control unit 600 may generate a shear modulus map representing the shear modulus of each point in the ROI.

Also, the control unit 600 may generate a shear modulus image 420 by mapping the shear modulus map with different colors according to the values of the shear modulus. For example, the ultrasound imaging apparatus 1000 may map with bluer color as the shear modulus is lower and map with redder color as the shear modulus is higher. Also, the control unit 600 may generate a shear modulus image 420 by mapping the shear modulus map with different brightness or chroma according to the values of the shear modulus. For example, the control unit 600 may map with lower brightness or chroma as the shear modulus is lower and map with higher brightness or chroma as the shear modulus is higher.

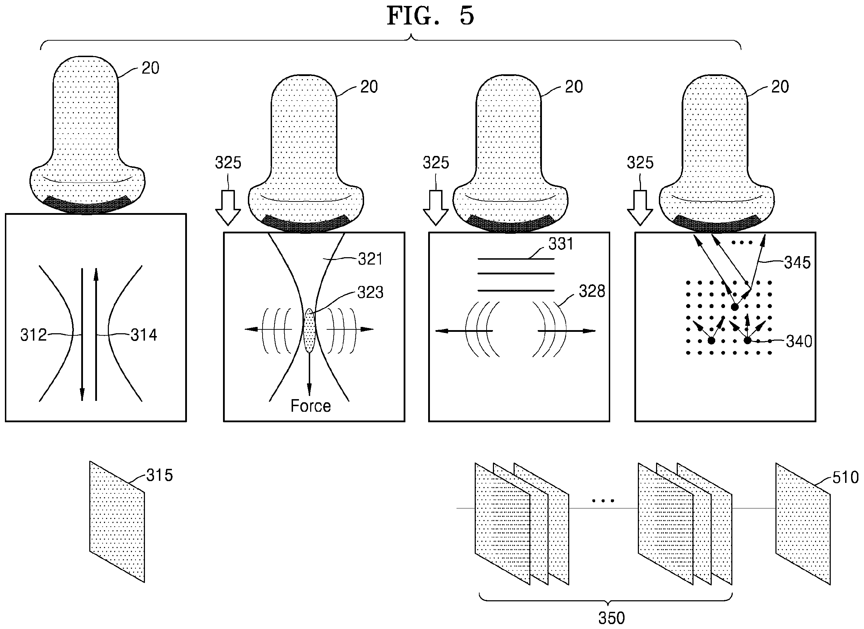

FIG. 5 is a diagram illustrating a method of calculating, by the ultrasound imaging apparatus 1000, a strain of the ROI, according to an exemplary embodiment.

After the first ultrasound echo signal 314 for the reference image 315 is received, the user may manually apply a force 325 to the object 10 through the probe 20 intentionally or unintentionally. Accordingly, the tissue in the ROI may be modified by the force 325 applied manually through the probe 20.

Referring to FIG. 5, the strain calculating module 620 may calculate a strain of the ROI, based on the reference image 315 and an image not including the shear wave 328, from among the ultrasound images generated based on the third ultrasonic echo pulse 345.

An image 510 generated after a reference time, from among the ultrasound images generated based on the third ultrasonic echo pulse 345, may be an image that does not include the shear wave 328 generated by the acoustic force. The image 510 generated after the reference time, from among the ultrasound images generated based on the third ultrasonic echo pulse 345, may be referred to as a strain sample image 510. The reference time may be preset based on the lateral width of the ROI and the density of the tissue in the ROI.

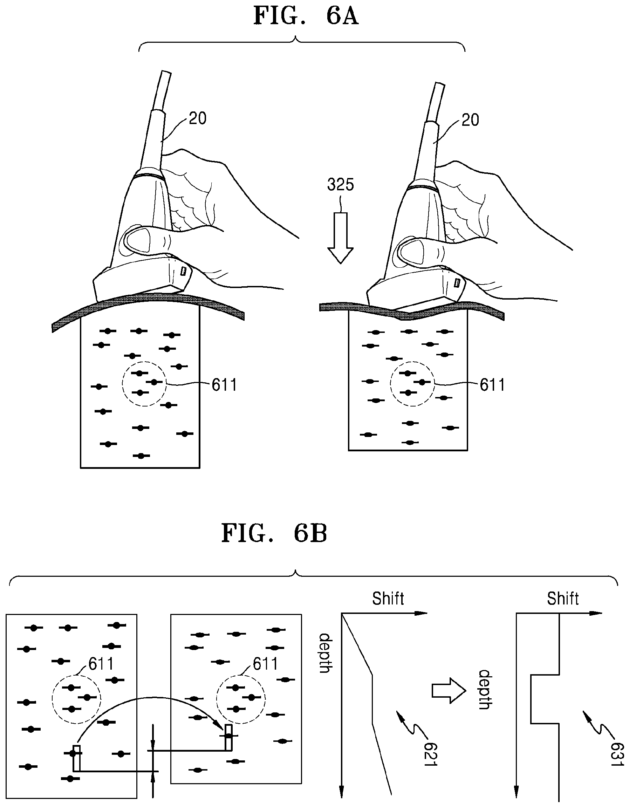

FIGS. 6a and 6b are diagrams illustrating a strain of a tissue generated by a force applied manually through the probe 20 of the ultrasound imaging apparatus 1000, according to an exemplary embodiment.

Referring to FIG. 6a, the user may manually apply a force 325 to the ROI, in which a tumor tissue is located, through the probe 20.

Referring to FIG. 6b, the control unit 600 may image the ROI before the force 325 is applied thereto and image the ROI after the force 325 is applied thereto. The strain calculating module 620 may calculate a strain of the ROI by comparing an image of the ROI captured before the force 325 is applied thereto and an image of the ROI captured after the force 325 is applied thereto.

A stiff tumor tissue 611 has a small displacement generated by the force 325. On the other hand, a relatively soft tissue has a large displacement generated by the force 325 because it may be easily compressed by the same force 325.

Thus, the strain calculating module 620 may calculate a strain of the at least one point in the ROI by calculating a displacement per unit length with respect to the at least one point in the ROI.

A strain "$\varepsilon$" may refer to a displacement per unit length. Also, the strain "$\varepsilon$" may be expressed as Equation 2 below.

Equation 2

$$\varepsilon = \frac{\triangle L}{L}$$

In Equation 2, $\varepsilon$ may denote the strain, L may denote the length of the tissue, and $\triangle_L$ may denote the displacement of the tissue.

Also, $\dfrac{\triangle L}{L}$ may change with respect to the strain of the partial tissue as expressed in Equation 3 below.

Equation 3

$$\sigma = \partial u_z / \partial z$$

In Equation 3, $u_z$ may denote the displacement in a z-axis direction. Thus, the strain may refer to a spatial derivative with respect to the displacement.

Referring to FIG. 6B, the strain calculating module 620 may calculate a depth-dependent displacement 621 of the tissue with respect to the ROI by comparing an ultrasound image captured before the force 325 is applied and an ultrasound image captured after the force 325 is applied.

As the depth-dependent displacement 621 of the tissue is calculated, the strain calculating module 620 may calculate a depth-dependent strain 631 of the tissue by differentiating the depth-dependent displacement 621 of the tissue in a depth direction.

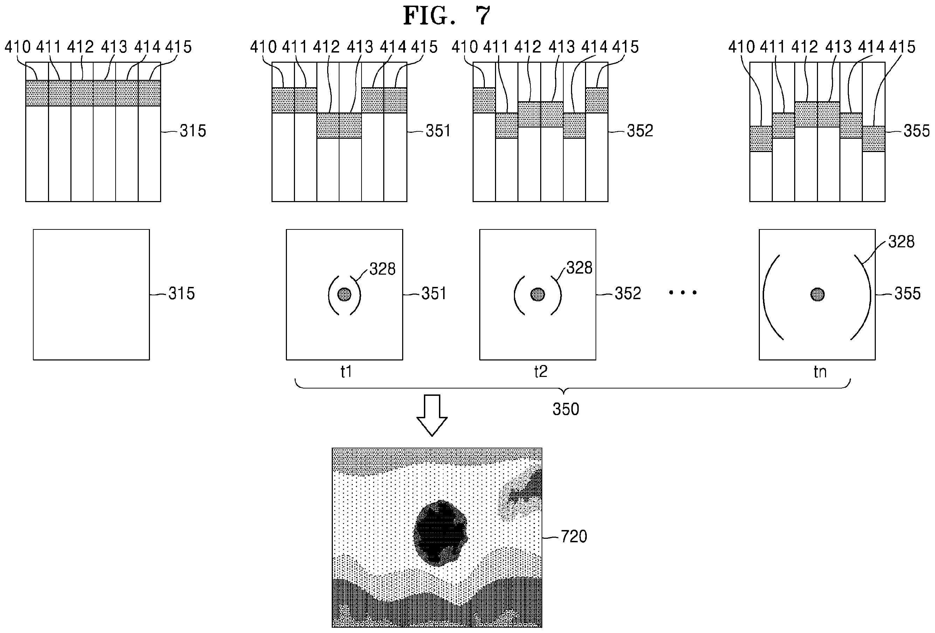

FIG. 7 is a diagram illustrating a method of calculating, by the ultrasound imaging apparatus 1000, a strain generated by a force applied manually through the probe 20, according to an exemplary embodiment.

Referring to FIG. 7, the strain calculating module 620 may calculate a strain of the at least one point in the ROI, based on the reference image 315 and the strain sample image 510.

The strain calculating module 620 may calculate the displacement of the tissue generated by a force applied manually through the probe 20.

For example, the strain calculating module 620 may detect the position of the partial tissue 412 in the reference image 315 from the strain sample image 510, based on a cross-correlation method. The strain calculating module 620 may calculate a displacement 710 of the partial tissue 412 in a force application direction, based on the detected position of the partial tissue 412. Also, the strain calculating module 620 may calculate the displacements of a plurality of partial tissues in the ROI in the force application direction.

The strain calculating module 620 may calculate a strain of the at least one point in the ROI by differentiating the calculated displacements of the plurality of partial tissues in the force application direction.

As the strain of the at least one point in the ROI is calculated, the control unit 600 may generate a strain map representing the strain of each point in the ROI.

Also, the control unit 600 may generate a strain image 720 by mapping the strain map with different colors according to the values of the strain. Also, the control unit 600 may generate a strain image 720 by mapping the strain map with different brightness or chroma according to the values of the strain.

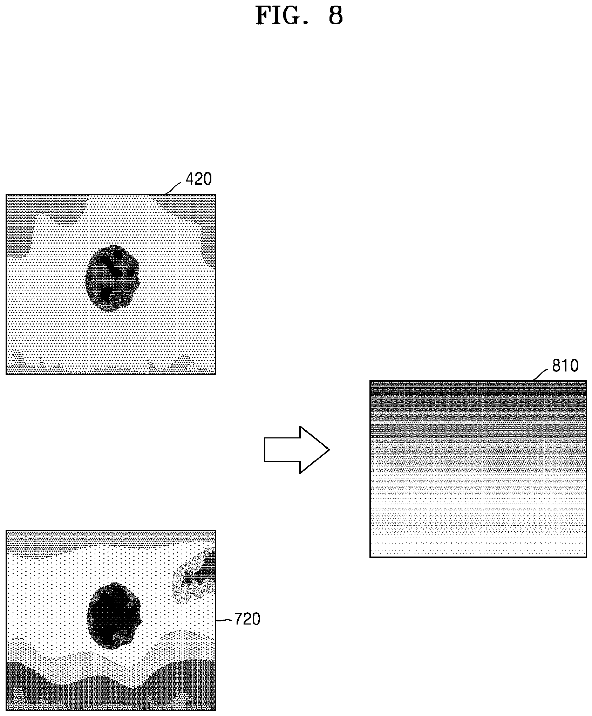

FIG. 8 is a diagram illustrating a method of calculating, by the ultrasound imaging apparatus 1000, a stress of the ROI, based on a shear modulus and a strain, according to an exemplary embodiment.

Referring to FIG. 8, the stress calculating module 630 may calculate a stress of the at least one point in the ROI, based on the strain and the elastic modulus of the at least one point in the ROI.

Also, the relationship between the stress and the strain may be expressed as Equation 4 below.

Equation 4

$$\sigma = E \cdot \varepsilon$$

In Equation 4, $\sigma$ may denote the stress, E may denote the elastic modulus, and $\varepsilon$ may denote the strain. Also, the stress "$\sigma$" may refer to a force per area.

Also, the relationship between the elastic modulus and the shear modulus may be expressed as Equation 5 below.

## Equation 5

$$E = 3G$$

In Equation 5, E may denote the elastic modulus and G may denote the shear modulus.

Thus, the stress may be expressed as Equation 6 below.

## Equation 6

$$\sigma = 3G \cdot \varepsilon$$

The stress calculating module 630 may calculate a stress applied to the at least one point in the ROI by calculating a product of the shear modulus and the strain of the at least one point in the ROI and tripling the product.

Also, the stress calculating module 630 may calculate a stress applied to each point in the ROI by calculating a product of the shear modulus and the strain of each point in the ROI and tripling the product, based on the shear modulus map and the strain map.

As the stress applied to the at least one point in the ROI is calculated, the control unit 600 may generate a stress map 810 representing the stress of each point in the ROI.

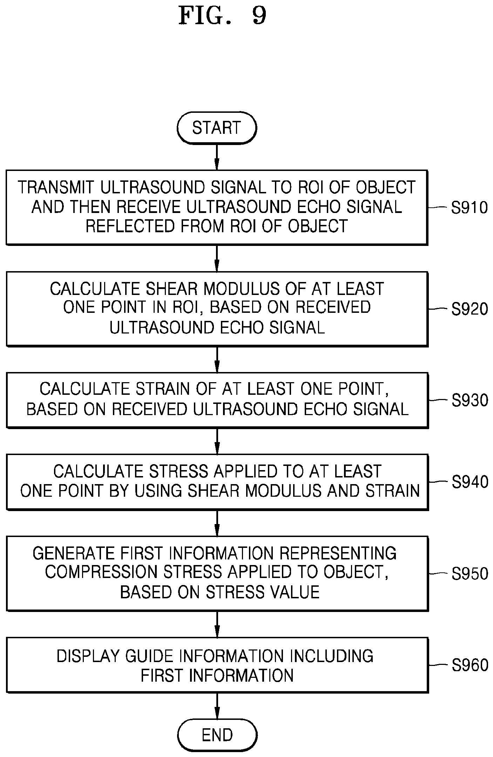

FIG. 9 is a flowchart illustrating a method of guiding, by the ultrasound imaging apparatus 1000, an operation of the user pressing the probe 20, according to an exemplary embodiment.

Referring to FIG. 9, in operation S910, the ultrasound imaging apparatus 1000 may transmit an ultrasound signal to the ROI of the object 10 and then receive an ultrasound echo signal reflected from the ROI of the object 10. In operation S920, the ultrasound imaging apparatus 1000 may calculate a shear modulus of the at least one point in the ROI, based on the received ultrasound echo signal. In operation S930, the ultrasound imaging apparatus 1000 may calculate a strain of the at least one point, based on the received ultrasound echo signal. In operation S940, the ultrasound imaging apparatus 1000 may calculate a stress applied to the at least one point by using the shear modulus and the strain. Operations S910 to S940 may correspond to operations S210 to S240 illustrated in FIG. 2.

In operation S950, the ultrasound imaging apparatus 1000 may generate first information representing a compression stress applied to the object 10, based on the stress.

The first information representing the compression stress applied to the object 10 may be information about a force of the user applied to the object 10.

The ultrasound imaging apparatus 1000 may calculate the force of the user applied to the object 10, based on the stress of the at least one point in the ROI.

In operation S960, the ultrasound imaging apparatus 1000 may display guide information including the first information.

The ultrasound imaging apparatus 1000 may display the information about the force of the user applied to the object 10, together with the shear modulus image.

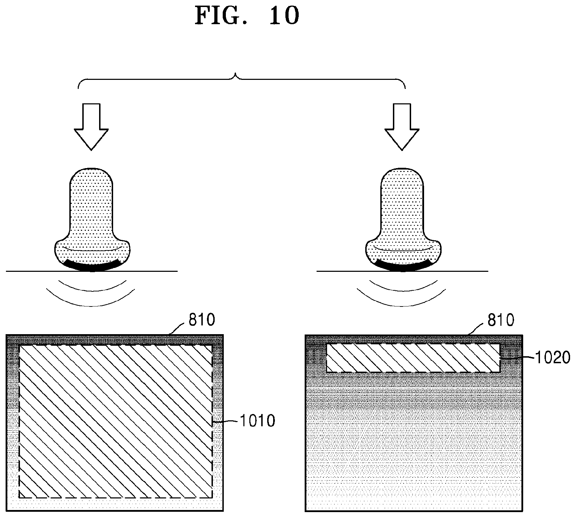

FIG. 10 is a diagram illustrating a method of calculating, by the ultrasound imaging apparatus 1000, a force of the user applied to the object 10 through the probe 20, according to an exemplary embodiment.

Referring to FIG. 10, the control unit 600 may calculate the force of the user applied to the object 10, based on the stress of the at least one point in the ROI.

The control unit 600 may calculate an average value of the stress on an entire ROI 1010 and determine the calculated average value as the force of the user applied to the object 10.

Also, the control unit 600 may calculate an average value of the stress on A region 1020 in the ROI, which is located within a predetermined distance from the surface of the object 10, and determine the calculated average value as the force of the user applied to the object 10.

Also, the control unit 600 may calculate an average value of the stress on a region in the ROI, which is located within a predetermined distance from the probe 20, and determine the calculated average value as the force of the user applied to the object 10.

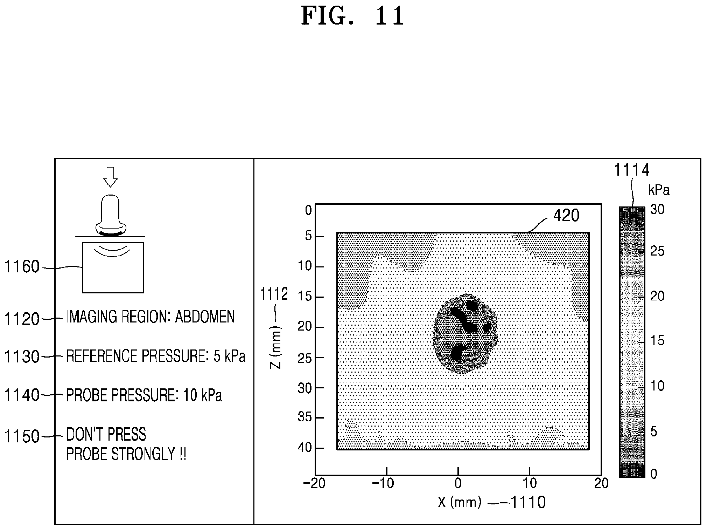

FIG. 11 is a diagram illustrating a method of guiding, by the ultrasound imaging apparatus 1000, an operation of the user pressing the probe 20 when capturing a shear modulus image, according to an exemplary embodiment.

Referring to FIG. 11, the display unit 230 of the ultrasound imaging apparatus 1000 may display guide information about a force of the user pressing the probe 20 when capturing a shear modulus image.

The stiffness of the tissue may be changed when the user presses the probe 20 with a pressure that is lower or higher than a reference pressure, when capturing a shear modulus image. A shear modulus image, which is captured when the stiffness of the tissue changes, may fail to quantitatively represent the original stiffness of the tissue.

The display unit 230 may guide the reference pressure to be applied through the probe 20, by displaying the guide information about the force of the user pressing the probe 20.

The display unit 230 may display the shear modulus image together with coordinate axes 1110 and 1112 representing the position of the at least one point in the ROI. Also, the display unit 230 may display the shear modulus image together with an image 1114 representing the value of the shear modulus corresponding to each color.

Also, the display unit 230 may display a region 1120 of the ROI of which the shear modulus has been calculated, a reference pressure 1130 with respect to the region 1120 of the ROI, a pressure 1140 of the probe 20 calculated based on the stress of the ROI, and a guidance text 1150 for guiding the force of the user pressing the probe 20.

The region 1120 of the ROI may be set by the user. Also, the reference pressure 1130 may be preset in the ultrasound imaging apparatus 1000, corresponding to a region of a human body.

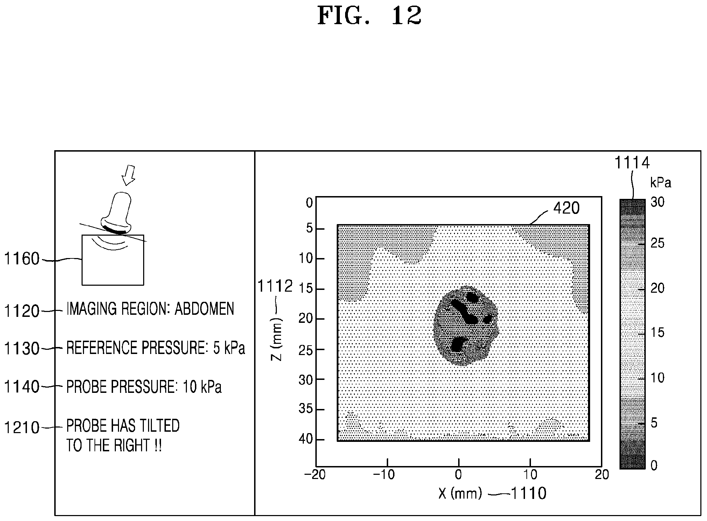

FIG. 12 is a diagram illustrating a method of guiding, by the ultrasound imaging apparatus 1000, an operation of the user pressing the probe 20 when capturing a shear modulus image, according to an exemplary embodiment.

Referring to FIG. 12, the display unit 230 may display guide information about the direction of the force of the user pressing the probe 20 when capturing a shear modulus image.

When capturing a shear modulus image, the user may tilt the probe 20 back and forth or right and left. When the probe 20 is tilted back and forth or right and left, the forces applied to partial regions in the ROI may be different from each other.

The display unit 230 may display information 1210 about the direction of the force of the user pressing the probe 20. For example, when the probe 20 is tilted to the right, the display unit 230 may display text 1210 representing the tilt direction and the current shape of the probe 20.

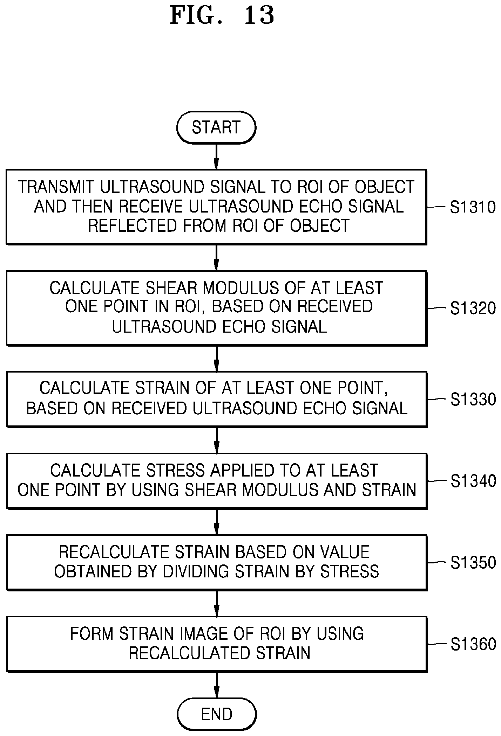

FIG. 13 is a flowchart illustrating a method of recalculating, by the ultrasound imaging apparatus 1000, a strain by using a stress, according to an exemplary embodiment.

Referring to FIG. 13, in operation S1310, the ultrasound imaging apparatus 1000 may transmit an ultrasound signal to the ROI of the object 10 and then receive an ultrasound echo signal reflected from the ROI of the object 10. In operation S1320, the ultrasound imaging apparatus 1000 may calculate a shear modulus of at least one point in the ROI, based on the received ultrasound echo signal. In operation S1330, the ultrasound imaging apparatus 1000 may calculate a strain of the at least one point, based on the received ultrasound echo signal. In operation S1340, the ultrasound imaging apparatus 1000 may calculate a stress applied to the at least one point by using the shear modulus and the strain. Operations S1310 to S1340 may correspond to operations S210 to S240 illustrated in FIG. 2.

In operation S1350, the ultrasound imaging apparatus 1000 may recalculate the strain, based on a value obtained by dividing the strain by the stress.

The ultrasound imaging apparatus 1000 may recalculate a value, which is obtained by dividing the strain of a first point in the ROI by the stress of the first point, as the strain of the first point.

Also, the ultrasound imaging apparatus 1000 may divide the ROI into at least one region, calculate an average stress of each of the at least one region, and recalculate the strain by dividing the strain of a first point in the ROI by the average stress of a region including the first point.

In operation S1360, the ultrasound imaging apparatus 1000 may form a strain image of the ROI by using the recalculated strain.

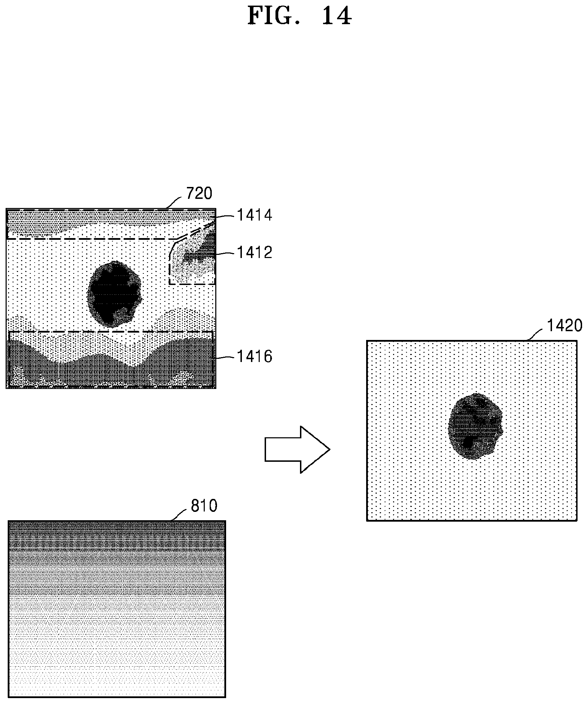

FIG. 14 is a diagram illustrating a method of recalculating, by the ultrasound imaging apparatus 1000, a strain by using a stress, according to an exemplary embodiment.

Referring to FIG. 14, the strain calculating module 620 may recalculate a strain of the ROI by using a calculated stress of each point in the ROI.

Referring to Equation 4, the strain of the tissue may increase as the stress applied to the tissue increases. Also, under the same stress, if the first tissue has a higher stiffness or a higher shear modulus than the second tissue, the first tissue may have a higher strain than the second tissue. Thus, assuming that the same stress is applied to the ROI, the strain of the tissue may refer to the stiffness of the tissue.

Assuming that the same stress is applied to the ROI, the strain image may be an image that is represented by measuring the stiffness of the ROI. However, the force applied to the probe 10 by the probe 20 may not uniformly act on the entire ROI. For example, the force applied by the probe 20 may greatly act on the tissue that is adjacent to the probe 20, and the force applied by the probe 20 may slightly act on the tissue that is distant from the probe 20. Also, the probe 20 may be obliquely pressed onto the object 10. Thus, the stiffness of the tissue represented in the strain image may be different from the original stiffness of the tissue.

Artifacts 1412 to 1416, which are generated because the force does not uniformly act on the entire ROI, may be observed in the strain image. The artifacts 1412 to 1416 may indicate that a general tissue having a low stiffness unlike a tumor is displayed differently from surrounding tissues since an excessive stress is applied to the general tissue.

The strain calculating module 620 may remove the artifacts by compensating for a non-uniform stress applied to the ROI by using the calculated stress. For example, the strain calculating module 620 may recalculate a value, which is obtained by dividing the strain of the first point in the ROI by the stress of the first point, as the strain of the first point.

The strain calculating module 620 may recalculate a strain map of the ROI by recalculating the strain of each point in the ROI. As the strain map of the ROI is recalculated, the control unit 600 may generate a strain image 1420, based on the recalculated strain map.

Also, the display unit 230 may display the strain image 1420 generated based on the strain calculated as a quantitative value.

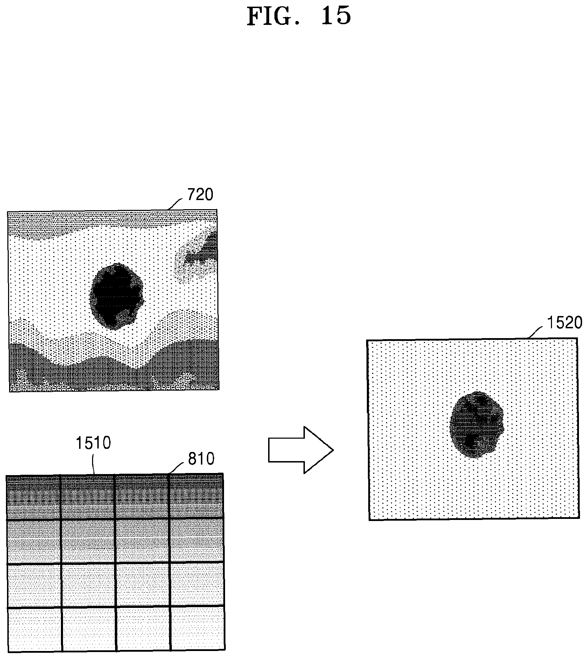

FIG. 15 is a diagram illustrating a method of recalculating, by the ultrasound imaging apparatus 1000, a strain by using a stress, according to another exemplary embodiment.

Referring to FIG. 15, the strain calculating module 620 may calculate an average stress of a plurality of partial regions and recalculate the strain of the partial region, based on the calculated average stress.

The stress does not change abruptly according to distance. Thus, the stresses applied to the adjacent tissues in the ROI may be regarded as being substantially identical to each other.

Accordingly, the control unit 600 may divide the ROI into a plurality of partial regions 1510 and calculate an average stress of the partial region 1510, based on the stress of each point in the partial region 1510. As the average stress of the partial region 1510 is calculated, the strain calculating module 620 may divide the strain of each point in the partial region 1510 by the average stress and recalculate the resulting value of the division as the strain of each point in the ROI. As the strain of each point in the ROI is recalculated, the control unit 600 may recalculate a strain map of the ROI.

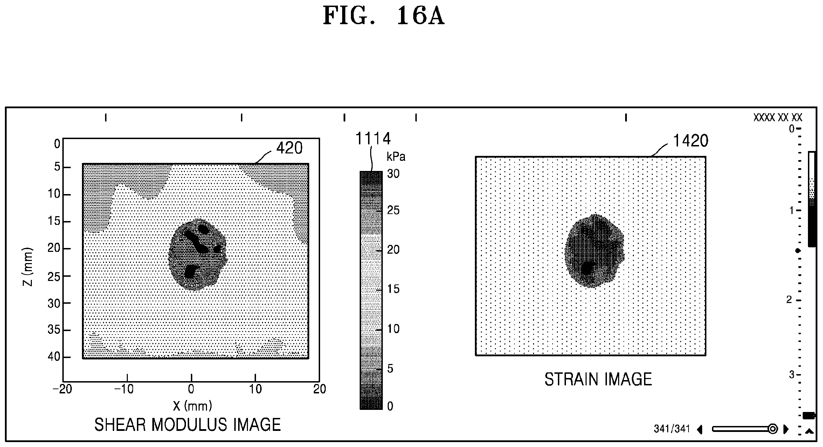

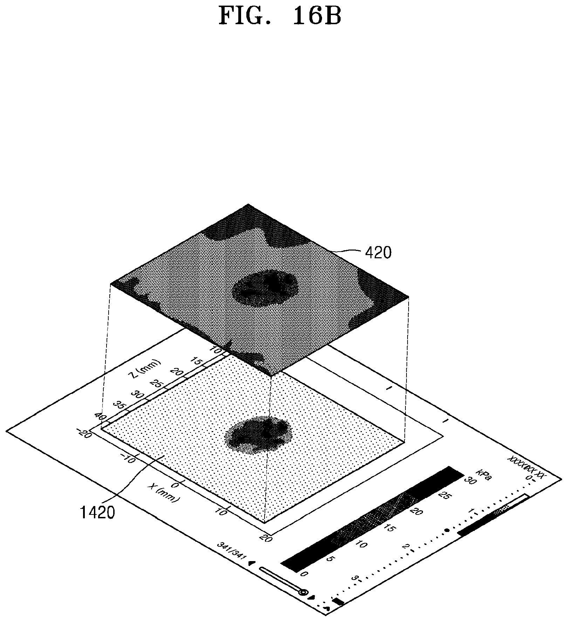

FIGS. 16a and 16b are diagrams illustrating a method of displaying, by the ultrasound imaging apparatus 1000, a shear modulus image and a strain image, according to an exemplary embodiment.

Referring to FIG. 16a, the display unit 230 may display the shear modulus image 420 and the strain image 1420 together.

The control unit 600 may acquire the shear modulus image 420 of the ROI by using the shear modulus of the at least one point in the ROI. Also, the control unit 600 may acquire the strain image 1420 of the ROI by using the strain of the at least one point in the ROI. Also, the display unit 230 may display the shear modulus image 420 and the strain image 1420 simultaneously on a screen.

Also, the display unit 230 may display the shear modulus image 420 and the strain image 1420 together with the B mode image, the M mode image, or the Doppler image.

Referring to FIG. 16b, the display unit 230 may display the shear modulus image 420 and the strain image 1420 in a superimposed manner.

For example, the display unit 230 may display the strain image 1420 and the shear modulus image 420 in a superimposed manner. In this case, the shear modulus image 420 may be displayed semitransparently so that the strain image 1420 may be projected through the shear modulus image 420.

Also, the display unit 230 may display the shear modulus image 420 and display the strain image 1420 on the shear modulus image 420 in a superimposed manner so that the shear modulus image 420 may be projected through the strain image 1420.

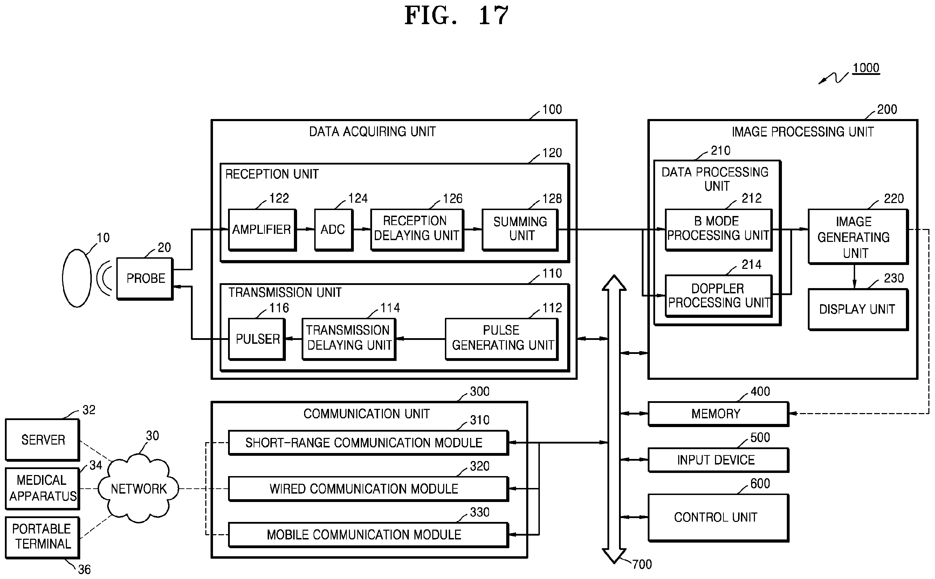

FIG. 17 is a block diagram illustrating a configuration of an ultrasound imaging apparatus 1000 according to an exemplary embodiment.

Referring to FIG. 17, the ultrasound imaging apparatus 1000 may include a data acquiring unit 100, a display unit 230, and a control unit 600 and may further include an image processing unit 200, a communication unit 300, a memory 400, and an input device 500, where the components stated above may be connected to one another via buses 700.

The ultrasound imaging apparatus 1000 may be embodied not only as a cart type ultrasound imaging apparatus, but also as a portable ultrasound imaging apparatus. Examples of the portable ultrasound imaging apparatus may include a picture archiving and communication system (PACS) viewer, a smart phone, a laptop computer, a personal digital assistant (PDA), and a tablet personal computer (PC); however, exemplary embodiments are not limited thereto.

A probe 20 transmits an ultrasound signal to an object 10 according to a driving signal applied from the data acquiring unit 100 and receives an echo signal reflected from the object 10. The probe 20 includes a plurality of transducers, and the plurality of transducers oscillate according to an electrical signal transmitted thereto and generate an ultrasonic wave, that is, acoustic energy. Also, the probe 20 may be connected to a main body of the ultrasound imaging apparatus 1000 by wire or wirelessly. According to exemplary embodiments, the ultrasound imaging apparatus 1000 may include a plurality of probes 20.

A transmission unit 110 supplies a driving signal to the probe 20 and includes a pulse generating unit 112, a transmission delaying unit 114, and a pulser 116. The pulse generating unit 112 generates pulses for forming transmission ultrasonic waves according to a predetermined pulse repetition frequency (PRF), and the transmission delaying unit 114 applies a delay time for determining transmission directionality to the pulses. The pulses to which a delay time is applied correspond to a plurality of piezoelectric vibrators included in the probe 20, respectively. The pulser 116 applies a driving signal (or a driving pulse) to the probe 20 at a timing corresponding to each pulse to which a delay time is applied.

A reception unit 120 generates ultrasound data by processing echo signals received from the probe 20 and may include an amplifier 122, an analog-digital converter (ADC) 124, a reception delaying unit 126, and a summing unit 128. The amplifier 122 amplifies echo signals in each channel, and the ADC 124 analog-digital converts the amplified echo signals. The reception delaying unit 126 applies delay times for determining reception directionality to the digital-converted echo signals, and the summing unit 128 generates ultrasound data by summing the echo signals processed by the reception delaying unit 126. Also, according to exemplary embodiments, the reception unit 120 may not include the amplifier 122. In other words, when the sensitivity of the probe 20 or the capability to process bits by the ADC 124 is enhanced, the amplifier 122 may be omitted.

The image processing unit 200 generates an ultrasound image by scan-converting ultrasound data generated by the data acquiring unit 100 and displays the ultrasound image. The ultrasound image may include not only a gray-scale image obtained by scanning the object 10 in an amplitude (A) mode, a brightness (B) mode, and a motion (M) mode, but also a Doppler image representing a motion of the object 10 by using a Doppler effect. The Doppler image may include a bloodstream Doppler image (also referred to as a color Doppler image) representing a flow of blood, a tissue Doppler image representing a motion of a tissue, and a spectral Doppler image representing a movement speed of the object 10 in a waveform.

A B mode processing unit 212 extracts B mode components from ultrasound data and processes the B mode components. An image generating unit 220 may generate an ultrasound image representing signal intensities as brightness, based on the B mode components extracted by the B mode processing unit 212.

Likewise, a Doppler processing unit 214 may extract Doppler components from ultrasound data, and the image generating unit 220 may generate a Doppler image representing a motion of the object 10 as colors or waveforms, based on the extracted Doppler components.

The image generating unit 220 according to an exemplary embodiment may generate a three-dimensional (3D) ultrasound image through volume-rendering of volume data and may also generate an elasticity image that visualizes the deformation of the object 10 due to a pressure. In addition, the image generating unit 220 may display various additional information in an ultrasound image by using texts and graphics. The generated ultrasound image may be stored in the memory 400.

The display unit 230 displays the generated ultrasound image. The display unit 230 may display not only an ultrasound image, but also various information processed by the ultrasound imaging apparatus 1000 on a screen via a graphic user interface (GUI). The ultrasound imaging apparatus 1000 may include two or more display units 230 according to exemplary embodiments.

The communication unit 300 is connected by wire or wirelessly to a network 30 to communicate with an external device or a server. The communication unit 300 may exchange data with a hospital server or other medical apparatuses in a hospital connected through a Picture Archiving and Communication System (PACS). Also, the communication unit 300 may perform data communication according to the Digital Imaging and Communications in Medicine (DICOM) standard.

The communication unit 300 may transmit and receive data related to diagnosis of the object 10, e.g., an ultrasound image, ultrasound data, and Doppler data of the object 10, via the network 30 and may also transmit and receive medical images obtained by other medical apparatuses, e.g., a computer tomography (CT) image, a magnetic resonance imaging (MRI) image, and an X-ray image. In addition, the communication unit 300 may receive information related to a diagnosis history or treatment schedule of a patient from a server and use the information to diagnose the object 10. In addition, the communication unit 300 may perform data communication not only with a server or a medical apparatus in a hospital, but also with a portable terminal of a doctor or a patient.

The communication unit 300 may be connected by wire or wirelessly to the network 30 to exchange data with a server 32, a medical apparatus 34, or a portable terminal 36. The communication unit 300 may include one or more components that enable communication with external devices, and may include, for example, a short-range communication module 310, a wired communication module 320, and a mobile communication module 330.

The short-range communication module 310 refers to a module for short-range communication within a predetermined distance. Examples of short-range communication techniques according to an exemplary embodiment may include wireless LAN, Wi-Fi, Bluetooth, Zigbee, Wi-Fi Direct (WFD), ultra wideband (UWB), infrared data association (IrDA), Bluetooth Low Energy (BLE), and near field communication (NFC); however, exemplary embodiments are not limited thereto.

The wired communication module 320 refers to a module for communication using electrical signals or optical signals. Examples of wired communication techniques according to an exemplary embodiment may include a twisted pair cable, a coaxial cable, an optical fiber cable, and an Ethernet cable.

The mobile communication module 330 transmits and receives wireless signals to and from at least one of a base station, an external terminal, and a server on a mobile communication network. Herein, the wireless signals may include voice call signals, video call signals, or various types of data for transmission and reception of text/ multimedia messages.

The memory 400 stores various data processed by the ultrasound imaging apparatus 1000. For example, the memory 400 may store medical data related to diagnosis of the object 10, such as ultrasound data and ultrasound images that are input or output and may also store algorithms or programs to be executed in the ultrasound imaging apparatus 1000.

The memory 400 may be embodied as any of various storage media such as a flash memory, a hard disk drive, and an electrically erasable programmable read-only memory (EEPROM). Also, the ultrasound imaging apparatus 1000 may utilize web storage or a cloud server that functions as the memory 400 online.

The input device 500 refers to a unit via which a user inputs data for controlling the ultrasound imaging apparatus 1000. The input device 500 may include hardware components, such as a keypad, a mouse, a touch panel, a touch screen, a track ball, and a jog switch. However, exemplary embodiments are not limited thereto, and the input device 50 may further include various other input units, such as an electrocardiogram measuring module, a respiration measuring module, a voice recognition sensor, a gesture recognition sensor, a fingerprint recognition sensor, an iris recognition sensor, a depth sensor, and a distance sensor.

The control unit 600 may control overall operations of the ultrasound imaging apparatus 1000. In other words, the control unit 600 may control operations among the probe 20, the data acquiring unit 100, the image processing unit 200, the communication unit 300, the memory 400, and the input device 500.

All or some of the probe 20, the data acquiring unit 100, the image processing unit 200, the communication unit 300, the memory 400, the input device 500, and the control unit 600 may be operated by software modules. However, exemplary embodiments are not limited thereto, and some of the components stated above may be operated by hardware modules. Also, at least one of the data acquiring unit 100, the image processing unit 200, and the communication unit 300 may be included in the control unit 600; however, exemplary embodiments are not limited thereto.

The exemplary embodiments may also be implemented in the form of a computer-readable recording medium including instructions executable by a computer, such as a program module executed by a computer. The computer-readable recording medium may be any available medium accessible by computers, examples of which may include a volatile recording medium, a nonvolatile recording medium, a removable recording medium, and an unremovable recording medium. Also, examples of the computer-readable recording medium may include a computer storage medium and a communication medium. Examples of the computer storage medium may include a volatile storage medium, a nonvolatile storage medium, a removable storage medium, and an unremovable storage medium that are implemented by any method or technology for storing information such as computer-readable instructions, data structures, program modules, or other data. Examples of the communication medium may include any information transmission medium including computer-readable instructions, data structures, program modules, other data of modulated data signals, or other transmission mechanisms.

The foregoing is illustrative of exemplary embodiments and is not to be construed as limiting thereof. Although the exemplary embodiments have been described above, those of ordinary skill in the art will readily appreciate that various modifications are possible in the exemplary embodiments without materially departing from the concepts and features of the exemplary embodiments. Therefore, it is to be understood that the exemplary embodiments described above should be considered in a descriptive sense only and not for purposes of limitation. For example, elements described as being combined may also be implemented in a distributed manner, and elements described as being distributed may also be implemented in a combined manner.

Therefore, the scope of the inventive concept is defined not by the detailed description of the exemplary embodiments but by the appended claims, and all modifications or differences within the scope should be construed as being included in the inventive concept.

In addition, other exemplary embodiments may also be implemented through computer-readable code/instructions in/on a medium, e.g., a computer-readable medium, to control at least one processing element to implement any of the above-described exemplary embodiments. The medium may correspond to any medium/media permitting the storage and/or transmission of the computer-readable code.

The computer-readable code may be recorded/transferred on a medium in a variety of ways, with examples of the medium including recording media, such as magnetic storage media (e.g., ROM, floppy disks, hard disks, etc.) and optical recording media (e.g., CD-ROMs, or DVDs), and transmission media such as Internet transmission media. Thus, the medium may be such a defined and measurable structure including or carrying a signal or information, such as a device carrying a bitstream according to one or more exemplary embodiments. The media may also be a distributed network, so that the computer-readable code is stored/transferred and executed in a distributed fashion. Furthermore, the processing element could include a processor or a computer processor, and processing elements may be distributed and/or included in a single device.

It should be understood that the exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope of the inventive concept as defined by the following claims.

**Claims**

1. An ultrasound imaging apparatus comprising:

   a data acquiring unit (100) configured to transmit an ultrasound signal to a region of interest (ROI) of an object and then receive an ultrasound echo signal reflected from the ROI of the object; and
   a control unit (600) configured to calculate a shear modulus of at least one point in the ROI and a strain of the at least one point, based on the received ultrasound echo signal, and calculate a stress applied to the at least one point by using the shear modulus and the strain,
   **characterized in that**
   the control unit (600) is further configured to divide the ROI into a plurality of regions, calculate an average stress of each of the plurality of regions, recalculate the strain by dividing the strain of a first point in the ROI by the average stress of a region including the first point, and generate a strain image (1420) based on the recalculated strain.

2. The ultrasound imaging apparatus of claim 1, wherein
the control unit (600) is further configured to generate first information representing a compression stress applied to the object, based on the stress, and
the ultrasound imaging apparatus further comprises a display unit (230) configured to display a user interface screen comprising the first information under the control of the control unit.

3. The ultrasound imaging apparatus of claim 1, wherein the control unit (600) is further configured to calculate a value, which is proportional to a product of the shear modulus and the strain, as the stress.

4. The ultrasound imaging apparatus of claim 1, wherein the control unit (600) is further configured to acquire a shear modulus map of the ROI by using the shear modulus of the at least one point, to acquire a strain imaging map of the ROI by using the strain of the at least one point, and to calculate the stress of the ROI, based on the shear modulus map and the strain imaging map.

5. The ultrasound imaging apparatus of claim 1, wherein
the ultrasound signal comprises a first ultrasound signal (312) used to generate a reference image (315), a second ultrasound signal (321) that is a focused ultrasonic pulse used to apply an acoustic force to the ROI, and a third ultrasound signal (331) used to calculate a displacement of the at least one point in the ROI, which is generated by the acoustic force and a pressure applied through an ultrasound probe (20), and
the ultrasound echo signal comprises a first ultrasound echo signal (314) corresponding to the first ultrasound signal and a third ultrasound echo signal corresponding to the third ultrasound signal, which are reflected from the ROI of the object.

6. The ultrasound imaging apparatus of claim 1, wherein
the control unit (600) is further configured to generate a shear modulus image of the ROI by using the shear modulus of the first point, and
the ultrasound imaging apparatus further comprises a display unit (230) configured to display the shear modulus image and the strain image simultaneously on a screen.

7. An ultrasound image processing method comprising:

transmitting an ultrasound signal to a region of interest (ROI) of an object and then receiving an ultrasound echo signal reflected from the ROI of the object (S210, S910, S1310);
calculating a shear modulus of at least one point in the ROI, based on the received ultrasound echo signal (S220, S920, S1320);
calculating a strain of the at least one point, based on the received ultrasound echo signal (S230, S930, S1330);
calculating a stress applied to the at least one point by using the shear modulus and the strain (S240, S940, S1340);
recalculating the strain based on a value obtained by dividing the calculated strain by the calculated stress (S1350); and
forming a strain image of the ROI by using the recalculated strain (S1360),
**characterized in that**
the recalculating of the strain comprises dividing the ROI into a plurality of regions, calculating an average stress of each of the plurality of regions, and recalculating the strain by dividing the strain of a first point in the ROI by the average stress of a region including the first point.

8. The ultrasound image processing method of claim 7, further comprising:

generating first information representing a compression stress applied to the object, based on the stress (S950); and
displaying a user interface screen comprising the first information (S960).

9. The ultrasound image processing method of claim 7, wherein the calculating of the stress applied to the at least one point by using the shear modulus and the strain comprises calculating a value, which is proportional to a product of the shear modulus and the strain, as the stress.

10. The ultrasound image processing method of claim 7, wherein the calculating of the stress applied to the at least one point by using the shear modulus and the strain comprises acquiring a shear modulus map of the ROI by using the shear modulus of the at least one point, acquiring a strain imaging map of the ROI by using the strain of the at least one point, and calculating the stress of the ROI, based on the shear modulus map and the strain imaging map.

11. The ultrasound image processing method of claim 7, further comprising:

generating a shear modulus image of the ROI by using the shear modulus of the first point; and displaying the shear modulus image and the strain image simultaneously on a screen.

## Patentansprüche

1. Ultraschallbildgebungsvorrichtung, die Folgendes umfasst:

    eine Datenerfassungseinheit (100), die dazu ausgelegt ist, ein Ultraschallsignal an eine Region von Interesse (ROI, Region Of Interest) eines Objekts zu senden und dann ein von der ROI des Objekts reflektiertes Signal zu empfangen; und
    eine Steuereinheit (600), die dazu ausgelegt ist, basierend auf dem empfangenen Ultraschallechosignal ein Schermodul wenigstens eines Punkts in der ROI und eine Belastung des wenigstens einen Punkts zu berechnen, und unter Verwendung des Schermoduls und der Belastung eine auf den wenigstens einen Punkt ausgeübte Spannung zu berechnen, **dadurch gekennzeichnet, dass** die Steuereinheit (600) weiterhin dazu ausgelegt ist, die ROI in eine Vielzahl von Regionen zu teilen, eine durchschnittliche Spannung für jede aus der Vielzahl von Regionen zu berechnen, die Belastung durch Teilen der Belastung eines ersten Punkts in der ROI durch die durchschnittliche Spannung einer den ersten Punkt enthaltenden Region neu zu berechnen, und basierend auf der neu berechneten Belastung ein Belastungsbild (1420) zu erzeugen.

2. Ultraschallbildgebungsvorrichtung nach Anspruch 1, wobei die Steuereinheit (600) weiterhin dazu ausgelegt ist, basierend auf der Spannung erste Informationen zu erzeugen, die eine auf das Objekt ausgeübte Druckspannung darstellen, und die Ultraschallbildgebungsvorrichtung weiterhin eine Anzeigeeinheit (230) umfasst, die dazu ausgelegt ist, einen Benutzeroberflächenbildschirm umfassend die ersten Informationen unter der Steuerung durch die Steuereinheit anzuzeigen.

3. Ultraschallbildgebungsvorrichtung nach Anspruch 1, wobei die Steuereinheit (600) weiterhin dazu ausgelegt ist, einen Wert, der proportional zu einem Produkt aus dem Schermodul und der Belastung ist, als die Spannung zu berechnen.

4. Ultraschallbildgebungsvorrichtung nach Anspruch 1, wobei die Steuereinheit (600) weiterhin dazu ausgelegt ist, eine Schermodulkarte der ROI unter Verwendung des Schermoduls des wenigstens einen Punkts zu erhalten, ein Belastungsbildkarte der ROI unter Verwendung der Belastung des wenigstens einen Punkts zu erhalten, und die Spannung der ROI basierend auf der Schermodulkarte und der Belastungsbildkarte zu berechnen.

5. Ultraschallbildgebungsvorrichtung nach Anspruch 1, wobei
das Ultraschallsignal Folgendes umfasst: ein erstes Ultraschallsignal (312), das zum Erzeugen eines Referenzbildes (315) verwendet wird, ein zweites Ultraschallsignal (321), bei dem es sich um einen fokussierten Ultraschallimpuls handelt, der zum Anlegen einer akustischen Kraft an die ROI verwendet wird, und ein drittes Ultraschallsignal (331), das verwendet wird, um eine Verschiebung des wenigstens einen Punkts in der ROI zu berechnen, die durch die akustische Kraft und einen durch eine Ultraschallsonde (20) ausgeübten Druck erzeugt wird, und das Ultraschallechosignal ein dem ersten Ultraschallsignal entsprechendes erstes Ultraschallechosignal (314) und ein dem dritten Ultraschallsignal entsprechendes drittes Ultraschallechosignal umfasst, die von der ROI des Objekts reflektiert werden.

6. Ultraschallbildgebungsvorrichtung nach Anspruch 1, wobei
die Steuereinheit (600) weiterhin dazu ausgelegt ist, unter Verwendung des Schermoduls des ersten Punkts ein Schermodulbild der ROI zu erzeugen, und
die Ultraschallbildgebungsvorrichtung weiterhin eine Anzeigeeinheit (230) umfasst, die dazu ausgelegt ist, das Schermodulbild und das Belastungsbild gleichzeitig auf einem Bildschirm anzuzeigen.

7. Verfahren zur Verarbeitung eines Ultraschallbildes, wobei das Verfahren Folgendes umfasst:

    Senden eines Ultraschallsignals an eine Region von Interesse (ROI, Region Of Interest) eines Objekts und dann Empfangen eines von der ROI des Objekts reflektierten Signals (S210, S910, S1310);
    Berechnen eines Schermoduls wenigstens eines Punkts in der ROI basierend auf dem empfangenen Ultraschallechosignal (S220, S920, S1320);
    Berechnen einer Belastung des wenigstens einen Punkts basierend auf dem empfangenen Ultraschallechosignal (S230, S930, S1330);
    Berechnen einer auf den wenigstens einen Punkt ausgeübten Spannung unter Verwendung des Schermoduls und der Belastung (S240, S940, S1340);
    Neuberechnen der Belastung basierend auf einem Wert, der erhalten wird mittels Teilen der berechneten Belastung durch die berechnete Spannung (S1350); und

Bilden eines Belastungsbildes der ROI unter Verwendung der neu berechneten Belastung (S1360),

**dadurch gekennzeichnet, dass**

das Neuberechnen der Belastung Folgendes umfasst: Teilen der ROI in eine Vielzahl von Regionen, Berechnen einer durchschnittlichen Spannung für jede aus der Vielzahl von Regionen, Neuberechnen der Belastung durch Teilen der Belastung eines ersten Punkts in der ROI durch die durchschnittliche Spannung einer den ersten Punkt enthaltenden Region.

8. Verfahren zur Verarbeitung eines Ultraschallbildes nach Anspruch 7, wobei das Verfahren weiterhin Folgendes umfasst:

Erzeugen erster Informationen, die eine auf das Objekt ausgeübte Druckspannung darstellen, basierend auf der Spannung (S950); und

Anzeigen eines Benutzeroberflächenbildschirms umfassend die ersten Informationen (S960).

9. Verfahren zur Verarbeitung eines Ultraschallbildes nach Anspruch 7, wobei das Berechnen der auf den wenigstens einen Punkt ausgeübten Spannung unter Verwendung des Schermoduls und der Belastung Folgendes umfasst: Berechnen eines Wertes, der proportional zu einem Produkt aus dem Schermodul und der Belastung ist, als die Spannung.

10. Verfahren zur Verarbeitung eines Ultraschallbildes nach Anspruch 7, wobei das Berechnen der auf den wenigstens einen Punkt ausgeübten Spannung unter Verwendung des Schermoduls und der Belastung Folgendes umfasst: Erhalten einer Schermodulkarte der ROI unter Verwendung des Schermoduls des wenigstens einen Punkts, Erfassen einer Belastungsbildkarte der ROI unter Verwendung der Belastung des wenigstens einen Punkts, und Berechnen der Spannung der ROI basierend auf der Schermodulkarte und der Belastungsbildkarte.

11. Verfahren zur Verarbeitung eines Ultraschallbildes nach Anspruch 7, wobei das Verfahren weiterhin Folgendes umfasst:

Erzeugen eines Schermodulbilds der ROI unter Verwendung des Schermoduls des ersten Punkts; und

gleichzeitiges Anzeigen des Schermodulbilds und des Belastungsbilds auf einem Bildschirm.

**Revendications**

1. Appareil échographique comprenant :

une unité d'acquisition de données (100) conçue pour émettre un signal ultrasonore vers une région d'intérêt (ROI, region of interest) d'un objet et ensuite recevoir un signal d'écho ultrasonore réfléchi par la ROI de l'objet, et

une unité de commande (600) conçue pour calculer un module de rigidité d'au moins un point de la ROI et une déformation de l'au moins un point, compte tenu du signal d'écho ultrasonore reçu, et pour calculer une contrainte à laquelle est soumis l'au moins un point au moyen du module de rigidité et de la déformation,

**caractérisé en ce que**

l'unité de commande (600) est conçue en outre pour diviser la ROI en une pluralité de régions, calculer une contrainte moyenne de chacune des régions de la pluralité de régions, recalculer la déformation en divisant la déformation d'un premier point de la ROI par la contrainte moyenne d'une région comportant le premier point, et produire une image illustrant la déformation (1420) compte tenu de la déformation recalculée.

2. Appareil échographique selon la revendication 1, dans lequel

l'unité de commande (600) est conçue en outre pour produire des premières informations représentatives d'une contrainte de compression à laquelle est soumis l'objet, compte tenu de la contrainte, et l'appareil échographique comprend en outre une unité d'affichage (230) conçue pour afficher un visuel d'interface utilisateur comprenant les premières informations, sous la commande de l'unité de commande.

3. Appareil échographique selon la revendication 1, dans lequel l'unité de commande (600) est conçue en outre pour calculer une valeur proportionnelle au produit du module de rigidité et de la déformation, formant la contrainte.

4. Appareil échographique selon la revendication 1, dans lequel l'unité de commande (600) est conçue en outre pour acquérir une carte des modules de rigidité de la ROI au moyen du module de rigidité de l'au moins un point, pour acquérir une carte de représentation de la déformation de la ROI au moyen de la déformation de l'au moins un point, et pour calculer la contrainte de la ROI, compte tenu de la carte des modules de rigidité et de la carte de représentation de la déformation.

5. Appareil échographique selon la revendication 1, dans lequel

le signal ultrasonore comprend un premier signal ultrasonore (312) servant à produire une image de référence (315), un deuxième signal ultrasonore (321) consistant en une impulsion ultrasonore focalisée servant à appliquer une force acoustique à la ROI, et un troisième signal ultrasonore (331) servant à calculer un déplacement de l'au moins un point de la ROI, produit sous l'effet de la force acoustique et de la pression appliquée par la sonde à ultrasons (20), et

le signal d'écho ultrasonore comprend un premier signal d'écho ultrasonore (314) correspondant au premier signal ultrasonore et un troisième signal d'écho ultrasonore correspondant au troisième signal ultrasonore, lesquels sont réfléchis par la ROI de l'objet.

6. Appareil échographique selon la revendication 1, dans lequel

l'unité de commande (600) est conçue en outre pour produire une image illustrant le module de rigidité de la ROI au moyen du module de rigidité du premier point, et

l'appareil échographique comprend en outre une unité d'affichage (230) conçue pour afficher l'image illustrant le module de rigidité et l'image illustrant la déformation simultanément sur un visuel.

7. Procédé de traitement d'image échographique comprenant :

l'émission d'un signal ultrasonore vers une région d'intérêt (ROI, region of interest) d'un objet, puis la réception d'un signal d'écho ultrasonore réfléchi par la ROI de l'objet (S210, S910, S1310),

le calcul d'un module de rigidité d'au moins un point de la ROI, compte tenu du signal d'écho ultrasonore reçu (S220, S920, S1320),

le calcul d'une déformation de l'au moins un point, compte tenu du signal d'écho ultrasonore reçu (S230, S930, S1330),

le calcul d'une contrainte à laquelle est soumis l'au moins un point au moyen du module de rigidité et de la déformation (S240, S940, S1340),

le recalcul de la déformation compte tenu d'une valeur obtenue en divisant la déformation calculée par la contrainte calculée (S1350), et

la formation d'une image illustrant la déformation de la ROI au moyen de la déformation recalculée (S1360),

**caractérisé en ce que**

le recalcul de la déformation comprend la division de la ROI en une pluralité de régions, le calcul d'une contrainte moyenne de chacune des régions de la pluralité de régions, et le recalcul de la déformation en divisant la déformation d'un premier point de la ROI par la contrainte moyenne d'une région comportant le premier point.

8. Procédé de traitement d'image échographique selon la revendication 7, comprenant en outre :

la production de premières informations représentatives d'une contrainte de compression à laquelle est soumis l'objet, compte tenu de la contrainte (S950), et

l'affichage d'un visuel d'interface utilisateur comprenant les premières informations (S960).

9. Procédé de traitement d'image échographique selon la revendication 7, dans lequel le calcul de la contrainte à laquelle est soumis l'au moins un point au moyen du module de rigidité et de la déformation comprend le calcul d'une valeur proportionnelle au produit du module de rigidité et de la déformation, formant la contrainte.

10. Procédé de traitement d'image échographique selon la revendication 7, dans lequel le calcul de la contrainte à laquelle est soumis l'au moins un point au moyen du module de rigidité et de la déformation comprend l'acquisition d'une carte des modules de rigidité de la ROI au moyen du module de rigidité de l'au moins un point, l'acquisition d'une carte de représentation de la déformation de la ROI au moyen de la déformation de l'au moins un point, et le calcul de la contrainte de la ROI, compte tenu de la carte des modules de rigidité et de la carte de représentation de la déformation.

11. Procédé de traitement d'image échographique selon la revendication 7, comprenant en outre :

la production d'une image illustrant le module de rigidité de la ROI au moyen du module de rigidité du premier point, et

l'affichage de l'image illustrant le module de rigidité et de l'image illustrant la déformation simultanément sur un visuel.

EP 3 197 366 B1

# FIG. 1

# FIG. 2

START

TRANSMIT ULTRASOUND SIGNAL TO ROI OF OBJECT AND
THEN RECEIVE ULTRASOUND ECHO SIGNAL
REFLECTED FROM ROI OF OBJECT — S210

CALCULATE SHEAR MODULUS OF AT LEAST
ONE POINT IN ROI, BASED ON RECEIVED
ULTRASOUND ECHO SIGNAL — S220

CALCULATE STRAIN OF AT LEAST ONE POINT,
BASED ON RECEIVED ULTRASOUND ECHO SIGNAL — S230

CALCULATE STRESS APPLIED TO AT LEAST ONE POINT
BY USING SHEAR MODULUS AND STRAIN — S240

END

FIG. 3

EP 3 197 366 B1

FIG. 4

EP 3 197 366 B1

# FIG. 5

EP 3 197 366 B1

# FIG. 6A

# FIG. 6B

FIG. 7

EP 3 197 366 B1

# FIG. 8

# FIG. 9

START

TRANSMIT ULTRASOUND SIGNAL TO ROI OF OBJECT AND THEN RECEIVE ULTRASOUND ECHO SIGNAL REFLECTED FROM ROI OF OBJECT — S910

CALCULATE SHEAR MODULUS OF AT LEAST ONE POINT IN ROI, BASED ON RECEIVED ULTRASOUND ECHO SIGNAL — S920

CALCULATE STRAIN OF AT LEAST ONE POINT, BASED ON RECEIVED ULTRASOUND ECHO SIGNAL — S930

CALCULATE STRESS APPLIED TO AT LEAST ONE POINT BY USING SHEAR MODULUS AND STRAIN — S940

GENERATE FIRST INFORMATION REPRESENTING COMPRESSION STRESS APPLIED TO OBJECT, BASED ON STRESS VALUE — S950

DISPLAY GUIDE INFORMATION INCLUDING FIRST INFORMATION — S960

END

# FIG. 10

FIG. 11

EP 3 197 366 B1

# FIG. 12

EP 3 197 366 B1

# FIG. 13

START

TRANSMIT ULTRASOUND SIGNAL TO ROI OF OBJECT
AND THEN RECEIVE ULTRASOUND ECHO SIGNAL
REFLECTED FROM ROI OF OBJECT —— S1310

CALCULATE SHEAR MODULUS OF AT LEAST
ONE POINT IN ROI, BASED ON RECEIVED
ULTRASOUND ECHO SIGNAL —— S1320

CALCULATE STRAIN OF AT LEAST ONE POINT,
BASED ON RECEIVED ULTRASOUND ECHO SIGNAL —— S1330

CALCULATE STRESS APPLIED TO AT LEAST
ONE POINT BY USING SHEAR MODULUS AND STRAIN —— S1340

RECALCULATE STRAIN BASED ON VALUE
OBTAINED BY DIVIDING STRAIN BY STRESS —— S1350

FORM STRAIN IMAGE OF ROI BY USING
RECALCULATED STRAIN —— S1360

END

# FIG. 14

# FIG. 15

## FIG. 16A

SHEAR MODULUS IMAGE

STRAIN IMAGE

1114

kPa

420

1420

341/341

EP 3 197 366 B1

# FIG. 16B

## FIG. 17

1000

**DATA ACQUIRING UNIT** 100

**RECEPTION UNIT** 120

AMPLIFIER 122 → ADC 124 → RECEPTION DELAYING UNIT 126 → SUMMING UNIT 128

**TRANSMISSION UNIT** 110

PULSER 116 ← TRANSMISSION DELAYING UNIT 114 ← PULSE GENERATING UNIT 112

10 — PROBE 20

**IMAGE PROCESSING UNIT** 200

**DATA PROCESSING UNIT** 210

B MODE PROCESSING UNIT 212

DOPPLER PROCESSING UNIT 214

IMAGE GENERATING UNIT 220

DISPLAY UNIT 230

**COMMUNICATION UNIT** 300

SHORT-RANGE COMMUNICATION MODULE 310

WIRED COMMUNICATION MODULE 320

MOBILE COMMUNICATION MODULE 330

SERVER 32

MEDICAL APPARATUS 34

PORTABLE TERMINAL 36

NETWORK 30

MEMORY 400

INPUT DEVICE 500

CONTROL UNIT 600

700

EP 3 197 366 B1

**REFERENCES CITED IN THE DESCRIPTION**

**Patent documents cited in the description**

- US 20090216119 A1